## Europäisches Patentamt
## European Patent Office
## Office européen des brevets

(11) Publication number: **0 049 720**
**B1**

(12) **EUROPEAN PATENT SPECIFICATION**

(45) Date of publication of patent specification: **13.08.86**

(51) Int. Cl.⁴: **A 61 F 2/02, C 08 L 23/00**

(21) Application number: **80303562.5**

(22) Date of filing: **09.10.80**

(54) Prosthesis comprising composite material.

(43) Date of publication of application:
**21.04.82 Bulletin 82/16**

(45) Publication of the grant of the patent:
**13.08.86 Bulletin 86/33**

(84) Designated Contracting States:
**CH DE FR LI**

(56) References cited:
**US-A-4 178 686**
**US-A-4 192 021**
**US-A-4 202 055**

(73) Proprietor: **NATIONAL RESEARCH DEVELOPMENT CORPORATION**
**101 Newington Causeway**
**London SE1 6BU (GB)**

(72) Inventor: **Bonfield, William**
**48 Harmer Greenlane**
**Digswell Welwyn Hertfordshire (GB)**
Inventor: **Bowman, Jeremy Archibald**
**43 Leas Drive**
**Iver Buckinghamshire (GB)**
Inventor: **Grynpas, Marc Daniel**
**21 Jules Terrace**
**Newton Massachusetts 02159 (US)**

(74) Representative: **Colmer, Stephen Gary et al**
**Patent Department National Research Development Corporation 101 Newington Causeway**
**London SE1 6BU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 049 720**

**Description**

This invention relates to prostheses; more particularly, this invention relates to prostheses comprising composite materials for use in orthopaedics, and to processes for preparing them.

This invention relates principally, but not exclusively, to endoprosthesis; that is, to the artificial replacement of parts of the anatomy internally resident in the human or animal body, principally bone.

Animal bone and ivory have, in the past, been employed in orthopaedic prosthesis. As with all natural materials, however, it is difficult to ensure the supply of these materials with adequate and predictable mechanical strengths. Moreover, since they comprise protein which is not of the patient's origin inflammatory reactions may arise because of their biological incompatibility. Accordingly, surgeons have resorted, and still resort, to a variety of synthetic engineering materials whose envisaged uses were usually remote from prosthesis.

One major example is in total hip replacement: in general, the femoral head is replaced with a cast alloy, usually an austenitic stainless steel or Co-Cr alloy, secured, usually by a polymethylmethacrylate bone cement, in the marrow cavity and seated in a high density polyethylene acetabular cup. The alloys used are selected primarily by virtue of their biocompatibility, resistance to corrosion, adequate fracture toughness and fatigue strength. They have proved satisfactory, but less than ideal, in such applications; as a result, attention has recently been devoted to the development of even more "bioinert" prosthetic materials with comparable strength properties. Examples include titanium alloys and ceramics, principally alumina. The brittle nature of ceramics, however, presents new problems for prosthesis.

All such synthetic prosthetic materials hitherto used, however, suffer from one major defect: the prosthetic material eventually becomes detached from the bone to which it was originally affixed. That is, hitherto, major surgical prosthetic operations have been intrinsically impermanent.

The present invention seeks to provide prostheses suitable for use in orthopaedics, especially as endoprostheses, which, in vivo, do not become detached from bone to which they are affixed.

According, therefore, to one aspect of the present invention there is provided a prosthesis which comprises a composite of a homo- or co-polyolefin having a weight average molecular weight ($\bar{M}_w$) greater than 100,000 with from 10% to 80% by volume of a particulate inorganic solid. Preferably, the polyolefin comprises polyethylene, polypropylene, polybutylene or a copolymer of ethylene and at least one of propylene, butylene and hexene, preferably linear polyethylene.

It is desirable that the polyolefin has a weight average molecular weight ($\bar{M}_w$) greater than 300,000. Desirably, the polyolefin has a $\bar{M}_w$ below 3,000,000, preferably below 1,000,000: above a $\bar{M}_w$ of 3,000,000 there are processing difficulties associated with forming and fabricating the composite.

The composite should have no more than 80%, preferably no more than 70%, especially from 20 to 60%, by volume of the particulate inorganic solid: above that value it is found that the particulate inorganic solid cannot be distributed homogeneously whereas at very low loadings the composite may be too compliant. The particulate inorganic solid component of the composite is present both to reinforce the composite and to enhance its stiffness, and is particularly suitable if its Young's modulus is from 50 to 150 GPa, preferably from 60 to 120 GPa. Suitable such inorganic solids are usually non-metallic and include ceramics, preferably calcium salts, for example calcium phosphates wherein the Ca:P ratio is from 1.0 to 1.5. Preferably, the calcium salt is a natural or synthetic hydroxyapatite or fluorapatite having a Ca:P ratio from 1.51 to 1.7. Other particulate inorganic solids include chalk, fly ash and silica.

The particulate inorganic solid, especially hydroxyapatite, may be used in the form of ground spherical particles, preferably wherein the particle size is from 90% being less than 100 μm to 0.05 μm, preferably from 90% being less than 50 μm to 0.1 μm. The inorganic solid may also be used in the form of acicular particles or platelets, the latter preferably having a maximum length of 500 μm and a maximum thickness of 20 μm. Mixtures of differing particulate inorganic solids may be used.

The composites in accordance with the present invention may be prepared by milling the polyolefin, suitably at a temperature above the softening point, for example for 200° to 260°C, preferably from 200° to 240°C, with the particulate inorganic solid which, desirably, has previously been dried, for example by heating at a temperature from 100° to 160°C for a period of 3 to 12 hours. If necessary, the inorganic solid is first ground to the requisite particle size. The polyolefin is usually incorporated first in the mill, the inorganic solid being added in small quantities until the desired volume fraction is attained. The milling time will depend on the charge incorporated but for 0.5 kg is typically from 1 to 2 hours. For higher volume fractions of inorganic solid it is often more convenient to use a two-stage milling adding, say, the inorganic solid to produce a 40% volume fraction "hide"; cooling this; remelting on the mill; and adding the remainder of the inorganic solid.

It is also possible to produce a composite by mixing in an extruder, and, again, re-extruding to obtain higher volume fractions of inorganic solid is often more convenient. A further method of producing the composite is by comminuting and finely admixing the components in the solid phase; sintering the mixture; and compacting, as by isostatic pressing, the sintered product.

The composites used in accordance with this invention have a Young's modulus from 2 to 40, preferably from 10 to 30 GPa; that is, they have a Young's modulus in the range of values recorded for compact bone. The composites may be oriented in a machine direction, but it is preferred that the ratio of

2

Young's modulus parallel to the machine direction and at right angles thereto does not exceed 3, preferably 2.

By "Young's modulus" is meant herein the dynamic modulus measured ultrasonically by the method of Bonfield and Grynpas in "Nature", *270*, No. 5636, pp. 453—454 (1977).

A prosthesis of this invention, preferably an endoprosthesis, particularly for the direct engagement of bone, may be a fracture fixation device, a jaw prosthesis or a prosthesis for the simple substitution of a local section of bone; especially, however, the endoprosthesis is a bone joint device, particularly for partial or total replacement of the hip or knee joints. In particular, the composite may be used to fabricate either or both of the femoral head and stem and the acetabular cup into which the head seats *in vivo*, although it may be used in the prosthesis of any joint affected by arthrosis.

The prosthesis may be fabricated by compression or injection moulding. In the former, the solid composite is remelted, suitably at a temperature from 190° to 250°C, preferably 200° to 230°C, charged to the prosthesis mould cavity under load until the cavity is filled, and then cooled under load. In the case of injection moulding, similar temperatures are used, but care must be taken to use an injection pressure and speed below that which causes degradation by scorching.

It will often prove desirable, especially with a polyolefin with a $\bar{M}_w$ <500,000, to γ-irradiate the fabricated implant prosthesis. Not only will this give better resistance to creep and environmental stress cracking, but it will also sterilise the prosthesis.

Where processing difficulties are encountered or might be expected, it is often desirable to use a polyolefin of lower $\bar{M}_w$ to form the composite more readily and then to irradiate. Throughout the specification and claims, molecular weights and particle sizes refer to these parameters as charged; these may change during blending and fabrication.

The following Example illustrates composites usable in the invention.

Polyolefin, as specified in Table I below, is melted and contained on and in the nip of a two roll mill. To the molten polyolefin are added aliquots of the inorganic particulate solid until the requisite volume fraction, as specified in Table I, is attached.

**0 049 720**

TABLE I

| | | Polyolefin (a) | Mill temp. (°C) | Milling time (hr.) | Inorganic particulate solid (b) | Ratio (a:b) | | Quality prepared (g) |
|---|---|---|---|---|---|---|---|---|
| 1. | | H020-54P[1] | 220 | 1 | $CaCO_3$ | 1:3 | by wt. | 200 |
| 2. | | " | " | " | " | 1:1 | " | 100 |
| 3. | | " | " | " | " | 1:2 | " | 150 |
| 4. | | " | " | " | " | 1:3 | " | 200 |
| 5. | | H020-54P | 225 | " | CBA[2] | 2:1 | by wt. | 300 |
| 6. | | " | " | " | " | 1:1 | " | 300 |
| 7. | | " | " | " | " | 1:2 | " | 300 |
| 8. | | " | 230 | 1.5 | " | 3:2[3] | by vol. | 450 |
| 9. | | " | " | " | " | 2:3 | " | 500 |
| 10. | | " | " | " | " | 1:4 | " | 500 |
| 11. | | H060-45P[4] | 250 | " | HAP[5] | 1:9 | " | 500 |
| 12. | | " | " | " | " | 1:4 | " | 400 |
| 13. | | " | " | " | " | 1:3 | " | 340 |
| 14. | | " | " | " | " | 1:3 | " | 550 |
| 15. | | " | " | " | " | 2:3 | " | 230 |

[1] A linear polyethylene (ex. BP Chemicals Ltd.) having an MFI 0.05, $\bar{M}_n$ 33,000 and $\bar{M}_w$ 312,000.

[2] Calcined bone ash (ex. Podmore) which is dried (6 hours at 140°C) and ground to nearly spherical particles of sub-micron size before adding to the polyolefin.

[3] Assuming a density of 3160 kg.m$^{-3}$.

[4] A linear ethylene hexene −1 copolymer (ex. BP Chemicals Ltd.) having a $\bar{M}_w$ about 240,000 with about 1—4 butyl branches per 1,000 chain carbon atoms.

[5] Synthetic hydroxyapatite in platelet form which is dried (8 hours at 140°C) before adding to the polyolefin.

TABLE II

| | | Polyolefin (a) | Inorganic particulate solid (b) | Ratio (a:b) | | Plaque weight (g) | Density (kgm$^{-3}$) | Press temperature (°C) |
|---|---|---|---|---|---|---|---|---|
| 16. | | H020-54P | $CaCO_3$ | 1:3 | by wt. | 17.4 | 1810 | 210 |
| 17. | | " | CBA | 2:1 | " | 11.0 | 1150 | " |
| 18. | | " | " | 2:1 | " | 11.0 | 1150 | " |
| 19. | | " | " | 1:1 | " | 13.1 | 1360 | " |
| 20. | | " | " | 1:1 | " | 13.1 | 1360 | " |
| 21. | | " | " | 1:1 | " | 12.7 | 1320 | " |
| 22. | | " | " | 1:1 | " | 12.5 | 1300 | 200 |

TABLE II (contd.)

| | | Polyolefin (a) | Inorganic particulate solid (b) | Ratio (a:b) | | Plaque weight (g) | Density (kgm⁻³) | Press temperature (°C) |
|---|---|---|---|---|---|---|---|---|
| | 23. | H020-54P | CBA | 1:1 | by wt. | 12.7 | 1320 | 200 |
| | 24. | " | " | 1:1 | " | 12.7 | 1320 | " |
| | 25. | " | " | 1:2 | " | 18.0 | 1870 | 220 |
| | 26. | " | " | 1:2 | " | 16.5 | 1720 | " |
| | 27. | " | " | 1:1 | " | 13.0 | 1360 | " |
| | 28. | " | " | 1:2 | " | 16.5 | 1720 | " |
| | 29. | " | " | 1:2· | " | 16.6 | 1730 | " |
| | 30. | " | " | 1:2 | " | 16.4 | 1710 | 210 |
| | 31. | " | " | 1:2 | " | 16.3 | 1700 | " |
| | 32. | " | " | 4:1 | by vol. | 13.7 | 1430 | 200 |
| | 33. | " | " | 4:1 | " | 13.7 | 1430 | " |
| | 34. | " | " | 4:1 | " | 13.3 | 1390 | " |
| | 35. | " | " | 4:1 | " | 13.0 | 1350 | " |
| | 36. | " | " | 3:2 | " | 19.9 | 2070 | " |
| | 37. | " | " | 3:2 | " | 19.6 | 2040 | " |
| | 38. | " | " | 3:2 | " | 19.3 | 2010 | " |
| | 39. | " | " | 2:3 | " | 26.8 | 2790 | " |
| | 40. | " | " | 2:3 | " | 25.0 | 2600 | " |
| | 41. | " | " | 2:3 | " | 25.2 | 2660 | " |
| | 42. | H060-45P | HAP | 9:1 | " | 15.5 | 1610 | " |
| | 43. | " | " | 9:1 | " | 15.0 | 1560 | " |
| | 44. | " | " | 4:1 | " | 14.1 | 1470 | " |
| | 45. | " | " | 4:1 | " | 14.9 | 1550 | " |
| | 46. | " | " | 3:2 | " | 20.0 | 2080 | " |
| | 47. | " | " | 3:2 | " | 18.4 | 1920 | " |

Further blends (see Table II) were made essentially as specified above. These were then compression moulded into plaques for use in determining mechanical properties of the blends.

**Claims**

1. A prosthesis which comprises a composite of a homo- or co-polyolefin having a weight average molecular weight ($\bar{M}_w$) greater than 100,000 with from 10% to 80% by volume of a particulate inorganic solid.

5

2. A prosthesis according to claim 1 wherein the polyolefin comprises polyethylene, polypropylene, polybutylene, or a copolymer of ethylene and at least one of propylene, butylene and hexene.

3. A prosthesis according to claim 2 wherein the polyolefin comprises linear polyethylene.

4. A prosthesis according to any preceding claim wherein the polyolefin has an $\bar{M}_w$ less than 1,000,000.

5. A prosthesis according to any preceding claim wherein the composite comprises up to 70% by volume of the particulate inorganic solid.

6. A prosthesis according to claim 5 wherein the composite comprises from 20% to 60% by volume of the particulate inorganic solid.

7. A prosthesis according to any preceding claim wherein the particulate inorganic solid has a Young's modulus from 50 to 150 GPa.

8. A prosthesis according to claim 7 wherein the particulate inorganic solid has a Young's modulus from 60 to 120 GPa.

9. A prosthesis according to any preceding claim wherein the particulate inorganic solid comprises a ceramic.

10. A prosthesis according to any preceding claim wherein the particulate inorganic solid comprises a calcium salt.

11. A prosthesis according to claim 10 wherein the calcium salt is a calcium phosphate in which the Ca:P ratio is from 1.0 to 1.5.

12. A prosthesis according to claim 10 wherein the calcium salt is a natural or synthetic hydroxyapatite or fluorapatite having a Ca:P ratio from 1.51 to 1.7.

13. A prosthesis according to any preceding claim wherein the inorganic solid is in the form of ground spherical particles.

14. A prosthesis according to claim 13 wherein the particle size is from 90% being less than 100 μm to 0.05 μm.

15. A prosthesis according to any of claims 1 to 12 wherein the inorganic solid is in the form of acicular particles or platelets.

16. A prosthesis according to claim 15 wherein the platelets have a maximum length of 500 μm and a maximum thickness of 20 μm.

17. A prosthesis according to any preceding claim wherein the composite has a Young's modulus from 2 GPa to 40 GPa.

18. A prosthesis according to claim 17 wherein the composite has a Young's modulus from 5 GPa to 30 GPa.

19. A prosthesis according to any preceding claim wherein the composite has been oriented in a machine direction.

20. A prosthesis according to any of claims 17 to 19 wherein, in the composite, the ratio $E_{||}:E_{|}$ is from 1 to 3.

21. A prosthesis according to any preceding claim which is a femoral prosthesis.

22. A prosthesis according to any preceding claim which has been γ-irradiated after fabrication.

**Patentansprüche**

1. Prothese, dadurch gekennzeichnet, daß sie einen Verbund aus einem Homo- oder Co-polyolefin mit einem Gewichtsdurchschnitt Molekulargewicht ($\bar{M}_w$) von mehr als 100.000 mit 10 Vol.-% bis 80 Vol.-% eines Teilchenförmigen anorganischen Feststoffes enthält.

2. Prothese nach Anspruch 1, dadurch gekennzeichnet, daß das Polyolefin Polyethylen, Polypropylen, Polybutylen oder ein Copolymeres von Ethylen und wenigstens einer der Verbindungen Propylen, Butylen und Hexen enthält.

3. Prothese nach Anspruch 2, dadurch gekennzeichnet, daß das Polyolefin lineares Polyethylen enthält.

4. Prothese nach einem der vorhergehenden Ansprüche, dadurch gekennzeichnet, daß das Polyolefin ein $\bar{M}_w$ von weniger als 1.000.000 hat.

5. Prothese nach einem der vorhergehenden Ansprüche, dadurch gekennzeichnet, daß der Verbund bis zu 70 Vol.-% des teilchenförmigen anorganischen Feststoffes enthält.

6. Prothese nach Anspruch 5, dadurch gekennzeichnet, daß der Verbund 20 Vol.-% bis 60 Vol.-% des teilchenförmigen anorganischen Feststoffes enthält.

7. Prothese nach einem der vorhergehenden Ansprüche, dadurch gekennzeichnet, daß der teilchenförmige anorganische Feststoff einen Youngschen Modul von 50 bis 150 GPa aufweist.

8. Prothese nach Anspruch 7, dadurch gekennzeichnet, daß der teilchenförmige anorganische Feststoff einen Youngschen Modul von 60 bis 120 GPa aufweist.

9. Prothese nach einem der vorhergehenden Ansprüche, dadurch gekennzeichnet, daß der teilchenförmige anorganische Feststoff ein Keramikmaterial aufweist.

10. Prothese nach einem der vorhergehenden Ansprüche, dadurch gekennzeichnet, daß der teilchenförmige anorganische Feststoff ein Kalziumsalz aufweist.

11. Prothese nach Anspruch 10, dadurch gekennzeichnet, daß das Kalziumsalz ein Kalziumphosphat ist, worin das Ca:P Verhältnis 1,0 bis 1,5 beträgt.

12. Prothese nach Anspruch 10, dadurch gekennzeichnet, daß das Kalziumsalz ein natürliches oder synthetisches Hydroxyapatit oder Fluorapatit mit einem Ca:P Verhältnis von 1,51 bis 1,7 ist.

13. Prothese nach einem der vorhergehenden Ansprüche, dadurch gekennzeichnet, daß der anorganische Feststoff in Form von gemahlenen sphärischen Teilchen vorliegt.

14. Prothese nach Anspruch 13, dadurch gekennzeichnet, daß die Teilchengröße für 90% weniger als 100 μm bis 0,05 μm beträgt.

15. Prothese nach einem der Ansprüche 1 bis 12, dadurch gekennzeichnet, daß der anorganische Feststoff in Form von nadelförmigen Teilchen oder Plättchen vorliegt.

16. Prothese nach Anspruch 15, dadurch gekennzeichnet, daß die Plättchen eine maximale Länge von 500 μm und eine maximale Dicke von 20 μm haben.

17. Prothese nach einem der vorhergehenden Ansprüche, dadurch gekennzeichnet, daß der Verbund einen Youngschen Modul von 2 GPa bis 40 GPa aufweist.

18. Prothese nach Anspruch 17, dadurch gekennzeichnet, daß der Verbund einen Youngschen Modul von 5 GPa bis 30 GPa aufweist.

19. Prothese nach einem der vorhergehenden Ansprüche, dadurch gekennzeichnet, daß der Verbund in einer Maschinenrichtung orientiert ist.

20. Prothese nach einem der Ansprüche 17 bis 19, dadurch gekennzeichnet, daß im Verbund das Verhältnis Youngscher $Modul_2$:Youngscher $Modul_1$ 1 bis 3 beträgt.

21. Prothese nach einem der vorhergehenden Ansprüche, dadurch gekennzeichnet, daß es sich um eine Oberschenkelprothese handelt.

22. Prothese nach einem der vorhergehenden Ansprüche, dadurch gekennzeichnet, daß sie nach der Herstellung γ-bestrahlt ist.


## Revendications

1. Prothèse qui comprend un matériau composite d'une homo- ou co-polyoléfine ayant un poids moléculaire moyen en poids ($\bar{M}_p$) supérieur à 100 000, avec de 10% à 80% en volume d'un solide minéral particulaire.

2. Prothèse selon la revendication 1, dans laquelle la polyoléfine comprend du polyéthylène, du polypropylène, du polybutylène ou un copolymère d'éthylène et d'au moins l'une des oléfines propylène, butylène et hexène.

3. Prothèse selon la revendication 2, dans laquelle la polyoléfine comprend du polyéthylène linéaire.

4. Prothèse selon l'une quelconque des revendications précédentes, dans laquelle la polyoléfine possède un $\bar{M}_p$ inférieur à 1 000 000.

5. Prothèse selon l'une quelconque des revendications précédentes, dans laquelle le matériau composite comprend jusqu'à 70% en volume du solide minéral particulaire.

6. Prothèse selon la revendication 5, dans laquelle le matériau composite comprend de 20% à 60% en volume du solide minéral particulaire.

7. Prothèse selon l'une quelconque des revendications précédentes, dans laquelle le solide minéral particulaire possède un module d'Young de 50 à 150 GPa.

8. Prothèse selon la revendication 7, dans laquelle le solide minéral particulaire possède un module d'Young de 60 à 120 GPa.

9. Prothèse selon l'une quelconque des revendications précédentes, dans laquelle le solide minéral particulaire comprend une matière céramique.

10. Prothèse selon l'une quelconque des revendications précédentes, dans laquelle le solide minéral particulaire comprend un sel de calcium.

11. Prothèse selon la revendication 10, dans laquelle le sel de calcium est un phosphate de calcium dans lequel le rapport Ca:P se situe entre 1,0 et 1,5.

12. Prothèse selon la revendication 10, dans laquelle le sel de calcium est une hydroxyapatite ou fluorapatite naturelle ou synthétique ayant un rapport Ca:P compris entre 1,51 et 1,7.

13. Prothèse selon l'une quelconque des revendications précédentes, dans laquelle le solide minéral est sous forme de particules sphériques broyées.

14. Prothèse selon la revendication 13, dans laquelle la dimension des particules est telle que 90% de celles-ci ont de moins de 100 μm à 0,05 μm.

15. Prothèse selon l'une quelconque des revendications 1 à 12, dans laquelle le solide minéral est sous forme de particules aciculaires ou de plaquettes.

16. Prothèse selon la revendication 15, dans laquelle les plaquettes ont une longueur maximale de 500 μm et une épaisseur maximale de 20 μm.

17. Prothèse selon l'une quelconque des revendications précédentes, dans laquelle le matériau composite possède un module d'Young de 2 GPa à 40 GPa.

18. Prothèse selon la revendication 17, dans laquelle le matériau composite possède un module d'Young de 5 GPa à 30 GPa.

19. Prothèse selon l'une quelconque des revendications précédentes, dans laquelle le matériau composite à été orienté dans un sens machine.

20. Prothèse selon l'une quelconque des revendications 17 à 19 dans laquelle, dans le matériau composite, le rapport $E_{II}:E_I$ est de 1 à 3.

21. Prothèse selon l'une quelconque des revendications précédentes, qui est une prothèse fémorale.

22. Prothèse selon l'une quelconque des revendications précédentes, qui, après sa fabrication, a été soumise à irradiation par des rayons gamma.